# EUROPÄISCHE PATENTSCHRIFT

(11) **EP 1 812 401 B1**
(45) Veröffentlichungstag und Bekanntmachung des Hinweises auf die Patenterteilung: **24.06.2015**
(21) Anmeldenummer: 05800634.7
(22) Anmeldetag: 04.11.2005
(51) Int. Cl.: C07D 221/18

(54) **VERFAHREN ZUR HERSTELLUNG VON PERYLEN-3,4-DICARBONSÄUREIMIDEN**
METHOD FOR PRODUCING PERYLENE-3,4-DICARBOXYLIC ACID IMIDES
PROCEDE DE PRODUCTION D'IMIDES D'ACIDE PERYLENE-3,4-DICARBOXYLIQUE

(30) Priorität: 09.11.2004 DE 102004054303
(43) Veröffentlichungstag der Anmeldung: 01.08.2007
(73) Patentinhaber: BASF SE, 67056 Ludwigshafen (DE)
(72) Erfinder: KÖNEMANN, Martin, 68163 Mannheim (DE); BLASCHKA, Peter, 67069 Ludwigshafen (DE); REICHELT, Helmut, 67435 Neustadt (DE)
(86) Internationale Anmeldenummer: PCT/EP2005/011823
(87) Internationale Veröffentlichungsnummer: WO 2006/050860

(56) Entgegenhaltungen:
- WO-A-96/22331
- LANGHALS H ET AL: "BALANCED DECARBOXYLATION OF AROMATIC POLYACIDS - A ONE-STEP SYNTHESIS OF PERYLENE-3,4-DICARBOXYLIC ANHYDRIDE" LIEBIGS ANNALEN/RECUEIL: ORGANIC AND BIOORGANIC CHEMISTRY - A EUROPEAN JOURNAL, VCH PUBLISHERS, FLORIDA, US, Bd. 3, 1997, Seiten 467-468, XP002060924 ISSN: 0947-3440

## Beschreibung

Die vorliegende Erfindung betrifft ein neues Verfahren zur Herstellung von Perylen-3,4-dicarbonsäureimiden, die am Imidstickstoffatom einen sterisch anspruchsvollen Substituenten tragen, durch Umsetzung eines Perylen-3,4:9,10-tetracarbonsäuredianhydrids mit einem sterisch gehinderten primären Amin in einem im wesentlichen wasserfreien Reaktionsmedium.

Perylen-3,4-dicarbonsäureimide eignen sich bekanntermaßen als Fluoreszenzfarbstoffe und Pigmente sowie als Zwischenprodukte für Fluoreszenzfarbstoffe, Pigmente, Pigmentadditive und IR-Absorber.

Die Herstellung der Perylen-3,4-dicarbonsäureimide (N-substituierte, im Perylengerüst substituierte oder unsubstituierte Imide) kann, wie in der WO-A-96/22331 beschrieben, durch Umsetzung der Perylen-3,4:9,10-tetracarbonsäuredianhydride mit primären Aminen in einem im wesentlichen wasserfreien Reaktionsmedium in Gegenwart einer tertiären stickstoffbasischen Verbindung als Lösungsmittel und eines Übergangsmetallkatalysators erfolgen.

Als geeignete tertiäre stickstoffbasische Verbindungen werden hier neben tertiären aliphatischen Aminen wie Trihexylamin und cyclischen Amiden wie N-Methylpyrrolidon insbesondere aromatische heterocyclische Imine wie Chinolin, Isochinolin und Chinaldin genannt. Mischungen dieser Verbindungen werden nicht erwähnt, und die beispielhaft beschriebenen Umsetzungen werden alle in Chinolin vorgenommen.

Bei dieser Umsetzung werden die Perylen 3,4-dicarbonsäureimide zwar in hohen Ausbeuten erhalten, jedoch ist ihre Reinheit insbesondere für die Anwendung als Zwischenprodukt für Fluoreszenzfarbstoffe und IR-Absorber in vielen Fällen nicht ausreichend, so daß das ebenfalls in der WO-A-96/22331 beschriebene Reinigungsverfahren (Überführen der Rohprodukte in N-Methylpyrrolidon-Addukte und Behandeln dieser Addukte mit Basen sowie gewünschtenfalls abschließendes Behandeln der wieder isolierten Produkte mit Säuren) an das Herstellungsverfahren angeschlossen werden muß. Dieses Reinigungsverfahren ist durch die vorzunehmenden Filtrationsschritte zeit- und damit kostenintensiv.

In der EP-A-657 436 ist die zur WO-A-96/22331 analoge Umsetzung unter Druck in Gegenwart von Wasser beschrieben. Die hier erhaltenen Ausbeuten liegen jedoch nur im Bereich von 10 bis 50%.

Der Erfindung lag die Aufgabe zugrunde, ein Verfahren bereitzustellen, nach dem Perylen-3,4-dicarbonsäureimide, die am Imidstickstoffatom sterisch anspruchsvolle Substistuenten tragen, auf vorteilhafte Weise in hoher Ausbeute und so hoher Reinheit zugänglich sind, daß eine zusätzliche Reinigung unterbleiben kann.

Demgemäß wurde ein Verfahren zur Herstellung von Perylen-3,4-dicarbonsäureimiden gefunden, die am Imidstickstoffatom einen sterisch anspruchvollen Substituenten tragen, und die allgemeine Formel II 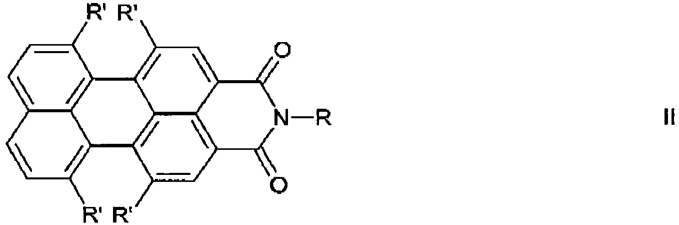 aufweisen, in der die Variablen folgende Bedeutung haben:
- R: C₃-C₂₄-Alkyl, das in 1-Position verzweigt ist, dessen Kohlenstoffkette durch eine oder mehrere Gruppierungen -O-, -S-, -NR⁴-, -CO- und/oder -SO₂- unterbrochen sein kann und das durch Carboxy, Sulfo, Hydroxy, Cyano, C₁-C₈-Alkoxy, C₃-C₈-Cycloalkyl, das ein bis vier Heteroatome im Ringsystem enthalten kann, und/oder C₆-C₁₀-Aryl, das ein bis drei Heteroatome im Ringsystem enthalten kann, ein- oder mehrfach substituiert sein kann;
Phenyl, das durch mindestens einen der Substituenten (a) bis (e) in einer ortho-Position substituiert ist und denselben oder einen oder mehrere davon verschiedene Substituenten (a) bis (e) in den weiteren Ringpositionen tragen kann und das ein bis zwei Heteroatome im Ring enthalten kann:
(a) C₁-C₂₄-Alkyl, dessen Kohlenstoffkette durch eine oder mehrere Gruppierungen -O-, -S-, -NR⁴-, -CO- und/oder -SO₂- unterbrochen sein kann und das durch Carboxy, Sulfo, Hydroxy, Cyano, Halogen, Nitro, C₁-C₆-Alkoxy, C₃-C₈-Cycloalkyl, das ein bis vier Heteroatome im Ringsystem enthalten kann, und/oder C₆-C₁₀-Aryl, das ein bis drei Heteroatome im Ringsystem enthalten kann, ein- oder mehrfach substituiert sein kann;
(b) C₁-C₂₄-Alkoxy oder C₁-C₂₄-Alkylthio, das jeweils durch C₁-C₆-Alkyl und/oder Phenyl ein- oder mehrfach substituiert sein kann;
(c) C₃-C₆-Cycloalkyl, das ein bis vier Heteroatome im Ringsystem enthalten kann, ungesättigte Bindungen enthalten kann und durch Carboxy, Sulfo, Hydroxy, Cyano, Halogen, Nitro, C₁-C₆-Alkoxy und/oder C₆-C₁₀-Aryl, das ein bis drei Heteroatome im Ringsystem enthalten kann, ein- oder mehrfach substituiert sein kann;
(d) C₆-C₁₀-Aryl, das ein bis drei Heteroatome im Ringsystem enthalten kann, an das weitere 5- bis 7-gliedrige, gesättigte, ungesättigte oder aromatische Ringe anneliert sein können und das durch C₁-C₁₂-Alkyl ein- oder mehrfach substituiert sein kann;
(e) Carboxy, Hydroxy, Cyano, Halogen, Nitro, -SO₂NR⁴₂; 1-Naphthyl, das in ortho-Position durch einen der für Phenyl genannten Substituenten (a) bis (e) substituiert ist und in den weiteren Ringpositionen denselben oder einen oder mehrere davon verschiedene Substituenten (a) bis (e) tragen kann und das ein bis drei Heteroatome im Ringsystem enthalten kann; 2-Naphthyl, das in beiden ortho-Positionen durch einen oder verschiedene der für Phenyl genannten Substituenten (a) bis (e) substituiert ist und in den weiteren Ringpositionen denselben oder einen oder mehrere davon verschiedene Substituenten (a) bis (e) tragen kann und das ein bis drei Heteroatome im Ringsystem enthalten kann;
- R': unabhängig voneinander Wasserstoff; Halogen; C₁-C₁₈-Alkyl; C₆-C₁₀-Aryl-oxy oder -Arylthio, das jeweils ein bis drei Heteroatome im Ringsystem enthalten kann und das jeweils durch C₁-C₁₀-Alkyl, C₁-C₆-Alkoxy, Cyano und/oder Carboxy substituiert sein kann;
- R⁴: Wasserstoff; C₁-C₁₂-Alkyl,
durch Umsetzung eines Perylen-3,4:9,10-tetracarbonsäuredianhydrids mit der allgemeinen Formel III 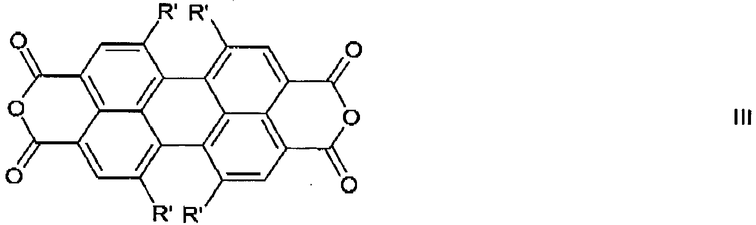 einem sterisch gehinderten primären Amin der allgemeinen Formel IV

R-NH₂ IV

in einem im wesentlichen wasserfreien Reaktionsmedium, welches dadurch gekennzeichnet ist, daß man die Umsetzung in Gegenwart eines tertiären Amins, eines Lösungsmittels auf Basis eines cyclischen Imins oder Amids als Lösungsmittel und einer Lewis-Säure als Katalysator vornimmt.

Wesentlich für das erfindungsgemäße Verfahren ist, daß zusätzlich zu dem cyclischen Imin als Lösungsmittel ein tertiäres Amin, also ein 3 Substituenten tragendes Amin (im folgenden als "aminischer Katalysator" bezeichnet), eingesetzt wird.

Als aminischer Katalysator eignen sich bevorzugt Amine der allgemeinen Formel I 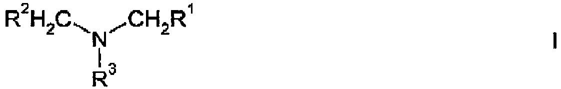 in der die Variablen folgende Bedeutung haben:
- R¹, R²: unabhängig voneinander
Wasserstoff;
C₁-C₂₃-Alkyl, dessen Kohlenstoffkette durch eine oder mehrere Gruppierungen -O-, -S- und/oder -NR⁴- unterbrochen sein kann und das durch Hydroxy, Cyano, Halogen, Nitro, C₆-C₁₀-Aryl, das ein bis drei Heteroatome im Ringsystem enthalten kann, und/oder C₄-C₁₂-Cycloalkyl, das ein bis vier Heteroatome im Ringsystem enthalten kann, substituiert sein kann;
C₄-C₁₂-Cycloalkyl, dessen Kohlenstoffkette durch eine oder mehrere Gruppierungen -O-, -S- und/oder -NR⁴- unterbrochen sein kann und das durch C₁-C₁₈-Alkyl, Hydroxy, Cyano, Halogen und/oder Nitro substituiert sein kann; C₆-C₁₀-Aryl, das durch C₁-C₁₈-Alkyl, Hydroxy, Cyano, Halogen und/oder Nitro substituiert sein kann;
zusammen einen die Gruppierung -CH₂-NR³-CH₂- enthaltenden 4- bis 9-gliedrigen gesättigten Ring, der durch weitere Gruppierungen -O-, -S- und/oder -NR⁴- unterbrochen sein kann und durch C₁-C₆-Alkyl, C₆-C₁₀-Aryl, Hydroxy, Cyano, Halogen und/oder Nitro substituiert sein kann;
- R³: C₄-C₂₄-Alkyl, dessen Kohlenstoffkette durch eine oder mehrere Gruppierungen -O-, -S- und/oder -NR⁴- unterbrochen sein kann und das durch Hydroxy, Cyano, Nitro, C₆-C₁₀-Aryl, das ein bis drei Heteroatome im Ringsystem enthalten kann, und/oder C₄-C₁₂-Cycloalkyl, das ein bis vier Heteroatome im Ringsystem enthalten kann, substituiert sein kann;
C₄-C₁₂-Cycloalkyl, dessen Kohlenstoffkette durch eine oder mehrere Gruppierungen -O-, -S- und/oder -NR⁴- unterbrochen sein kann und das durch C₁-C₁₈-Alkyl, Hydroxy, Cyano, Halogen und/oder Nitro substituiert sein kann; C₆-C₁₀-Aryl, das ein bis drei Heteroatome im Ringsystem enthalten kann und durch C₁-C₁₈-Alkyl, Hydroxy, Cyano und/oder Nitro substituiert sein kann;
- R¹, R²: und R³ miteinander unter Ausbildung eines gesättigten, bicyclischen, die Gruppierung 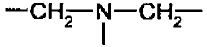 und gegebenenfalls weitere Gruppierungen -O-, -S- und/oder -NR⁴- enthalten-den 8- bis 12-gliedrigen Ringsystems verbunden;
- R⁴: Wasserstoff; C₁-C₁₂-Alkyl.

Besonders bevorzugt sind aminische Katalysatoren der Formel I, in der die Variablen folgende Bedeutung haben:
- R¹, R²: unabhängig voneinander
Wasserstoff;
C₁-C₁₁-Alkyl, das durch Hydroxy substituiert sein kann;
zusammen einen die Gruppierung -CH₂-NR³-CH₂- enthaltenden 4- bis 9-gliedrigen gesättigten Ring, der durch weitere Gruppierungen -NR⁴- unterbrochen sein kann;
- R³: C₄-C₁₂-Alkyl, das durch Hydroxy substituiert sein kann; Pyridyl;
- R¹, R²: und R³ miteinander unter Ausbildung eines bicyclischen, die Gruppierung 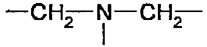 und ein weiteres Stickstoffatom enthaltenden 8-gliedrigen Ringsystems verbunden;
- R⁴: Wasserstoff; C₁-C₁₂-Alkyl.

Besonders bevorzugt wird das erfindungsgemäße Verfahren zur Herstellung von Perylen-3,4-dicarbonsäureimiden der allgemeinen Formel II angewendet, in der die Variablen folgende Bedeutung haben:
- R: C₃-C₂₄-Alkyl, das in 1-Position verzweigt ist;
Phenyl, das in beiden ortho-Positionen durch sekundäre C₃-C₁₂-Alkylreste oder in einer ortho-Position durch einen tertiären C₄-C₁₂-Alkylrest substituiert ist und das an den anderen Positionen durch C₁-C₁₂-Alkyl, C₆-C₁₀-Aryl und/oder Halogen substituiert sein kann;
- R': Wasserstoff; Halogen; C₆-C₁₀-Aryloxy oder -Arylthio, das jeweils durch C₁-C₁₀-Alkyl, C₁-C₆-Alkoxy, Cyano und/oder Carboxy substituiert sein kann.

Als Beispiele für die in den Formeln I bis IV genannten Reste R, R' und R¹ bis R⁴ sowie deren Substituenten seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2-und 3-Methoxypropyl, 2- und 3-Ethoxypropyl, 2- und 3-Propoxypropyl, 2- und 3-Butoxy-propyl, 2- und 4-Methoxybutyl, 2- und 4-Ethoxybutyl, 2- und 4-Propoxy-butyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxade-cyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthio-ethyl, 2-Butylthioethyl, 2- und 3-Methylthiopropyl, 2- und 3-Ethylthiopropyl, 2- und 3-Propylthiopropyl, 2-und 3-Butylthiopropyl, 2- und 4-Methylthiobutyl, 2- und 4-Ethylthio-butyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Chlor, Brom und Iod;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethyl-cycloheptyl, 3- und 4-Propylcycloheptyl, 3- und 4-Isopropylcycloheptyl, 3- und 4-Butyl-cycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propyl-cyclooctyl; 3- und 4-Hydroxycyclohexyl, 3- und 4- Nitrocyclohexyl und 3- und 4-Chlorcyclohexyl;
2-Dioxanyl, 4-Morpholinyl, 2- und 3-Tetrahydrofuryl, 1-, 2- und 3-Pyrrolidinyl und 1-, 2-, 3- und 4-Piperidyl;
Phenyl, 2-Naphthyl, 2-, 3- und 4-Pyridyl, 2-, 4- und 5-Pyrimidyl, 6-Chinaldyl, 3-, 5-, 6-und 8-Chinolinyl, und 1- und 5-Isochinolyl;
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Tri-methoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3-und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Im folgenden werden Beispiele für geeignete aminische Katalysatoren im einzelnen aufgeführt, wobei die Amine besonders geeignet, deren Siedepunkt oberhalb der Reaktionstemperatur liegt:
- Umsetzungsprodukte von Ammoniak, Monoalkylaminen, Dialkylaminen, Arylaminen, Alkylarylaminen und cyclischen Dialkylaminen, deren Alkylreste vorzugsweise 2 bis 24, insbesondere 2 bis 12 C-Atome und deren Arylreste bevorzugt 6 bis 10, vor allem 6 C-Atome, aufweisen, mit Alkylenoxiden und Arylenoxiden, vorzugsweise C₂-C₁₂-Alkylenoxiden, insbesondere C₂-C₆-Alkylenoxiden, vor allem C₂-C₃-Alkylenoxiden, wobei die Umsetzungsprodukte von Dialkylaminen und Monoalkylaminen mit Alkylenoxiden besonders bevorzugt sind:
   N,N-Diethylethanolamin, N,N-Dibutylethanolamin, N,N-Dipentylethanoiamin, N,N-Dihexylethanolamin, N,N-Dioctylethanolamin, N,N-Didodecylethanolamin, N,N-Dioctadecylethanolamin, Triethanolamin, 1-Diethylaminopropan-2-ol, 1-Diethylaminobutan-2-ol, 1-Diethylaminopentan-2-ol, 1-Diethylaminododecan-2-ol, 1-Diethylaminooctadecan-2-ol, 1(Dipropylaminopropan-2-ol, 1-Dipropylaminobutan-2-ol, 1-Dipropylaminopentan-2-ol, 1-Dipropylaminododecan-2-ol, 1-Dipropylaminooctadecan-2-ol, 1-Dibutylaminopropan-2-ol, 1-Dibutylaminobutan-2-ol, 1-Dibutylaminopentan-2-ol, 1-Dibutylaminododecan-2-ol und 1-Dibutylamino-octadecan-2-ol;
   N,N-(Di-2-hydroxyethyl)butylamin, N,N-(Di-2-hydroxyethyl)hexylamin, N,N-(Di-2-hydroxyethyl)octylamin, N,N-(Di-2-hydroxyprop-2-yl)butylamin, N,N-(Di-2-hydroxyprop-2-yl)hexylamin und N,N-(Di-2-hydroxyprop-2yl)octylamin;
   N-Phenyldiethanolamin, N-(p-Tolyl)diethanolamin, N-Phenyldi(2-propanol)amin, N-(p-Tolyl)di(2-propanol)amin, N-Phenyldi(2-butanol)amin und N-(p-Tolyl)di(2-butanol)amin;
- Trialkylamine, deren Alkylreste 3 bis 24, insbesondere 3 bis 12 C-Atome aufweisen:
   Tripropylamin, Tributylamin, Tripentylamin, Trihexylamin, Triheptylamin, Trioctylamin, Trinonylamin, Tridecylamin, Tridodecylamin, Trioctadecylamin, Diethylpropylamin, Dipropylbutylamin, Dibutylpentylamin, Dihexylheptylamin, Dimethyl-(2-ethylhexyl)amin und Dimethyl-(3-propylheptyl)amin;
- Cyclische Trialkylamine:
   N-Alkylmorpholine, wie N-Methylmorpholin; 1,4-Diazabicyclo[2,2,2]octan (DABCO); N,N'-Dialkylpiperazine, wie N,N'-Dimethylpiperazin; N,N'-Diarylpiperazine, wie N,N'-Diphenylpiperazin; N-Alkyl-N'-arylpiperazine, wie N-Phenyl-N'-methylpiperazin;
- Dialkylarylamine:
   Dimethylanilin, Diethylanilin, Dipropylanilin und Dibutylanilin;
- Dialkylaminoheteroaromaten, vorzugsweise 4-(N,N-(Di-C₁-C₄-alkyl)amino)pyridine, und mit cyclischen Aminogruppen substituierte Heteroaromaten:
   4-(N,N-Dimethylamino)pyridin (DMAP), 4-(N,N-Diethylamino)pyridin und 4-(N,N-Dipropylamino)pyridin; 4-Pyrrolidin-1-ylpyridin und 4-Piperidin-1-ylpyridin.

Dabei sind die Umsetzungsprodukte von Mono- und Dialkylaminen mit Ethylenoxid und/oder Propylenoxid, cyclische Trialkylamine und Dialkylaminoheteroaromaten und mit cyclischen Aminogruppen substituierte Heteroaromaten als aminische Katalysatoren bevorzugt.

Besonders bevorzugt sind C₃-C₆-Dialkylethanolamine, z.B. Dibutylethanolamin, C₃-C₁₀-Alkyldiethanol- und vor allem -dipropanolamine, z.B. Octyldipropanolamin, 1,4-Diazabicyclo[2,2,2]octan und 4-(N,N-(Di-C₁-C₄-alkyl)amino)pyridine, z.B. 4-(N,N-Dimethylamino)pyridin, 4- Pyrrolidin-1-ylpyridin und 4-Piperidin-1-ylpyridin.

Selbstverständlich können auch Mischungen der aminischen Katalysatoren verwendet werden.

In der Regel werden 0,01 bis 4 mol, vorzugsweise 0,5 bis 2 mol, aminischer Katalysator je mol Perylen-3,4:9,10-tetracarbonsäuredianhydrid eingesetzt.

Als Lösungsmittel kommen beim erfindungsgemäßen Verfahren cyclische imine oder Amide zum Einsatz. Geeignet sind neben cyclischen Amiden wie N-Methylpyrrolidon vor allem cyclische Imine wie Chinolin, Isochinolin und Chinaldin, wobei Chinolin als Lösungsmittel bevorzugt ist. Selbstverständlich können auch Lösungsmittelmischungen eingesetzt werden.

Die Menge an Lösungsmittel Ist an sich nicht kritisch und liegt üblicherweise bei 2 bis 20 kg, vorzugsweise bei 1 bis 8 kg, je kg Parylen-3,4:9,10-tetracarbonsäuredianhydrid.

Als weiterer Katalysator wird beim erfindungsgemäßen Verfahren eine Lewis-Säure eingesetzt. Bevorzugt handelt es sich dabei um einen Übergangsmetallkatalysator auf der Basis von Eisen und vor allem Zink oder Kupfer. Besonders bevorzugt sind die anorganischen und die organischen Salze dieser Metalle. Selbstverständlich kann man auch Mischungen dieser Katalysatoren verwenden.

Beispiele für bevorzugte Salze sind Kupfer(I)oxid, Kupfer(II)oxid, Kupfer(I)chlorid, Kupfer(II)acetat, Zinkacetat und Zinkpropionat.

In der Regel kommen 0,1 bis 10, vorzugsweise 0,5 bis 3 Moläquivalente Lewis-SäureKatalysator, bezogen auf das Perylen-3,4:9,10-tetracarbonsäuredianhydrid, zum Einsatz.

Beim erfindungsgemäßen Verfahren wird in einem im wesentlichen wasserfreien Reaktionsmedium gearbeitet. D.h., das bei der Umsetzung freiwerdende Reaktionswasser wird kontinuierlich abdestilliert oder durch einen Trägergasstrom, z.B. einen Stickstoffstrom, ausgetrieben und alle Reaktionskomponenten werden in möglichst wasserfreier Form eingesetzt. Es ist jedoch auch möglich, in den Reaktionskomponenten enthaltenes Wasser, z. B. Hydratwasser von Katalysatorsalzen, während der Reaktion abzudestillieren.

Als primäre Amine werden beim erfindungsgemäßen Verfahren sterisch gehinderte primäre Amine eingesetzt, insbesondere die Amine der Formel IV.

In der Regel werden je mol Perylen-3,4:9,10-tetracarbonsäuredianhydrid 1 bis 6 mol, bevorzugt 1,5 bis 4 mol, primäres Amin eingesetzt.

Die Reaktionstemperatur beträgt im allgemeinen 120 bis 250°C, vorzugsweise 180 bis 250°C.

Es empfiehlt sich, unter Verwendung einer Schutzgasatmosphäre (z.B. Stickstoff) zu arbeiten.

Üblicherweise ist die erfindungsgemäße Umsetzung in 2 bis 40 h, insbesondere in 5 bis 30 h beendet.

Verfahrenstechnisch geht man beim erfindungsgemäßen Verfahren zweckmäßigerweise wie folgt vor:
Man gibt Perylen-3,4:9,10-tetracarbonsäuredianhydxrid, Lewis-Säure-Katalysator, primäres Amin und aminischen Katalysator in das Lösungsmittel, spült die Apparatur mit Stickstoff und erhitzt das Gemisch unter Rühren auf die Reaktionstemperatur. Dabei wird entstehendes Reaktionswasser sowie eventuell in den eingesetzten Reaktionskomponenten enthaltenes Wasser abdestilliert. Nach etwa 5 bis 30 ständigem Rühren bei der Reaktionstemperatur kühlt man auf 50 bis 80°C ab und verdünnt durch Zugabe eines primäres aliphatischen Alkohols, z.B. Methanol, Ethanol, Propanol oder Ethylenglykol.

Das auf diese Weise ausgefällte Perylen-3,4-dicarbonsäureimid kann durch Filtration isoliert werden. Zur Entfernung eines metallhaltigen Lewis-Säure-Katalysators kann es in anorganischer Säure (z.B. 5 bis 20 gew.-%iger Salzsäure oder Schwefelsäure) bei 50 bis 80°C ausgerührt werden. Diese Behandlung kann auch in Kombination mit einem primären aliphatisches Alkohol, wie Ethanol, vorgenommen werden. Das abfiltrierte Produkt wird abschließend üblicherweise mit Ethanol sowie Wasser gewaschen und getrocknet.

Die auf diese Welse erhaltenen Perylen-3,4-dicarbonsäureimide weisen so hohe Wertgehalte auf (in der Regel > 94%), daß sie keiner weiteren Reinigung unterzogen werden müssen und direkt für alle gewünschten Anwendungszwecke eingesetzt werden können.

Das erfindungsgemäße Verfahren eignet sich hervorragend zur Herstellung aller Perylen-3,4-dicarbonsäureimide, die sterisch gehinderte Substituenten am Imidstickstoffatom tragen. Es können sowohl im Rylenkern substituierte als auch unsubstituierte Perylen-3,4-dicarbonsäureimide in hohen Ausbeuten (üblicherweise 45 bis 65%) hergestellt werden.

### Beispiele

### Beispiel 1

Zu einer Mischung von 1500 ml Chinolin, 585 g (3 mol) 2,6- Diisopropylanilin und 294 g (1,2 mol) N,N-(Di-2-hydroxyprop-2-yl)octylamin wurden 400 g (1 mol) Perylen-3,4:9,10-tetracarbonsäuredianhydrid und 400 g (1,8 mol) Zinkacetat unter starkem Rühren zugegeben. Dann wurde die Mischung auf 210°C erhitzt und 30 h bei dieser Temperatur gerührt. Dabei destillierten etwa 50 ml Wasser ab.

Nach Abdestillieren des Chinolins wurde der Rückstand zweimal mit 2000 g 20 gew.-%iger Schwefelsäure bei 70°C aufgerührt, abfiltriert und zuerst mit Wasser und dann so lange mit Ethanol gewaschen, bis das Filtrat farblos war, und danach getrocknet.

Es wurden 258 g N-(2,6-Diisopropylphenyl)perylen-3,4-dicarbonsäureimid mit einem Wertgehalt von 95% erhalten, was einer Ausbeute von 51% entspricht.

### Beispiel 2

Zu einer Mischung von 1500 ml Chinolin, 585 g (3 mol) 2,6- Diisopropylanilin und 200 g (1,2 mol) N,N-Dibutylethanolamin wurden 400 g (1 mol) Perylen-3,4:9,10-tetracarbonsäure-dianhydrid und 395 g (1,8 mol) wasserfreies Zinkacetat unter starkem Rühren zugegeben. Dann wurde die Mischung auf 215°C erhitzt und 28 h bei dieser Temperatur gerührt. Dabei destillierten etwa 50 ml Wasser ab.

Nach Abdestillieren eines Teils des Chinolins, Abkühlen auf 75°C und Verdünnen mit 1400 ml Ethanol wurde das ausgefällte Produkt abfiltriert und zunächst mit etwa 2 I Ethanol und dann wiederholt mit insgesamt 4400 g 16 gew.-%iger Schwefelsäure bei 70°C gerührt und abschließend mit Wasser neutral gewaschen und im Vakuum getrocknet.

Es wurden 247 g N-(2,6-Diisopropylphenyl)perylen-3,4-dicarbonsäureimid mit einem Wertgehalt von 96% erhalten, was einer Ausbeute von 50% entspricht.

### Beispiel 3

Zu einer Mischung von 100 ml Chinolin, 58,5 g (0,3 mol) 2,6-Diisopropylanilin und 13,4 g (0,12 mol) 1,4-Diazabicyclo[2.2.2]octan und 14,3 g (0,18 mol) Kupfer(II)oxid wurden 40 g (0,1 mol) Perylen-3,4:9,10-tetracarbonsäuredianhydrid unter starkem Rühren zugegeben. Dann wurde die Mischung unter einem leichten Stickstoffstrom zum Austreiben des Reaktionswassers 20 h auf 200°C erhitzt.

Nach Abdestillieren des Chinolins unter leicht vermindertem Druck wurde der Rückstand in einer Mischung von 40 ml 20 gew.-%iger Schwefelsäure und etwa 200 ml Ethanol bei 50°C aufgerührt. Nach Abkühlen auf Raumtemperatur wurde das Produkt abfiltriert, nochmals bei 50°C in frischer Schwefelsäure/Ethanol-Mischung aufgerührt, erneut abfiltriert und wiederholt bei Raumtemperatur mit Ethanol gewaschen. Nach abschließendem Waschen mit Wasser wurde das Produkt im Vakuum getrocknet.

Es wurden 24,1 g N-(2,6-Diisopropylphenyl)perylen-3,4-dicarbonsäureimid mit einem Wertgehalt von 94% erhalten, was einer Ausbeute von 47% entspricht.

## Patentansprüche

1. Verfahren zur Herstellung von Perylen-3,4-dicarbonsäureimiden, die am Imidstickstoffatom einen sterisch anspruchsvollen Substituenten tragen und die allgemeine Formel II 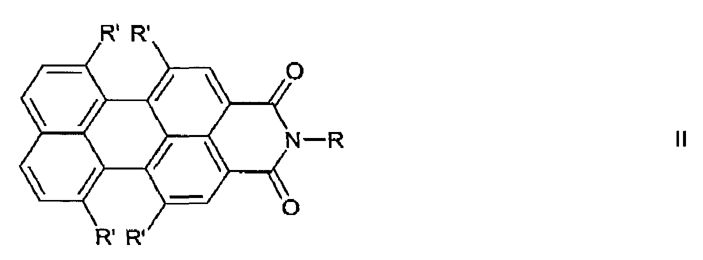 aufweisen, in der die Variablen folgende Bedeutung haben:
R C₃-C₂₄-Alkyl, das in 1-Position verzweigt ist, dessen Kohlenstoffkette durch eine oder mehrere Gruppierungen -O-, -S-, -NR⁴-, -CO- und/oder -SO₂- unterbrochen sein kann und das durch Carboxy, Sulfo, Hydroxy, Cyano, C₁-C₈-Alkoxy, C₃-C₈-Cycloalkyl, das ein bis vier Heteroatome im Ringsystem enthalten kann, und/oder C₆-C₁₀-Aryl, das ein bis drei Heteroatome im Ringsystem enthalten kann, ein- oder mehrfach substituiert sein kann; Phenyl, das durch mindestens einen der Substituenten (a) bis (e) in einer ortho-Position substituiert ist und denselben oder einen oder mehrere davon verschiedene Substituenten (a) bis (e) in den weiteren Ringpositionen tragen kann und das ein bis zwei Heteroatome im Ring enthalten kann:
(a) C₁-C₂₄-Alkyl, dessen Kohlenstoffkette durch eine oder mehrere Gruppierungen -O-, -S-, -NR⁴-, -CO- und/oder -SO₂- unterbrochen sein kann und das durch Carboxy, Sulfo, Hydroxy, Cyano, Halogen, Nitro, C₁-C₆-Alkoxy, C₃-C₈-Cycloalkyl, das ein bis vier Heteroatome im Ringsystem enthalten kann, und/oder C₆-C₁₀-Aryl, das ein bis drei Heteroatome im Ringsystem enthalten kann, ein- oder mehrfach substituiert sein kann;
(b) C₁-C₂₄-Alkoxy oder C₁-C₂₄-Alkylthio, das jeweils durch C₁-C₆-Alkyl und/oder Phenyl ein- oder mehrfach substituiert sein kann;
(c) C₃-C₈-Cycloalkyl, das ein bis vier Heteroatome im Ringsystem enthalten kann, ungesättigte Bindungen enthalten kann und durch Carboxy, Sulfo, Hydroxy, Cyano, Halogen, Nitro, C₁-C₆-Alkoxy und/oder C₆-C₁₀-Aryl, das ein bis drei Heteroatome im Ringsystem enthalten kann, ein- oder mehrfach substituiert sein kann;
(d) C₆-C₁₀-Aryl, das ein bis drei Heteroatome im Ringsystem enthalten kann, an das weitere 5- bis 7-gliedrige, gesättigte, ungesättigte oder aromatische Ringe anneliert sein können und das durch C₁-C₁₂-Alkyl ein- oder mehrfach substituiert sein kann;
(e) Carboxy, Hydroxy, Cyano, Halogen, Nitro, -SO₂NR⁴₂; 1-Naphthyl, das in ortho-Position durch einen der für Phenyl genannten Substituenten (a) bis (e) substituiert ist und in den weiteren Ringpositionen denselben oder einen oder mehrere davon verschiedene Substituenten (a) bis (e) tragen kann und das ein bis drei Heteroatome im Ringsystem enthalten kann;
2-Naphthyl, das in beiden ortho-Positionen durch einen oder verschiedene der für Phenyl genannten Substituenten (a) bis (e) substituiert ist und in den weiteren Ringpositionen denselben oder einen oder mehrere davon verschiedene Substituenten (a) bis (e) tragen kann und das ein bis drei Heteroatome im Ringsystem enthalten kann;
R' unabhängig voneinander Wasserstoff; Halogen; C₁-C₁₈-Alkyl; C₆-C₁₀-Aryloxy oder -Arylthio, das jeweils ein bis drei Heteroatome im Ringsystem enthalten kann und das jeweils durch C₁-C₁₀-Alkyl, C₁-C₆-Alkoxy, Cyano und/oder Carboxy substituiert sein kann;
R⁴ Wasserstoff; C₁-C₁₂-Alkyl,
durch Umsetzung eines Perylen-3,4:9,10-tetracarbonsäuredianhydrids der allgemeinen Formel III 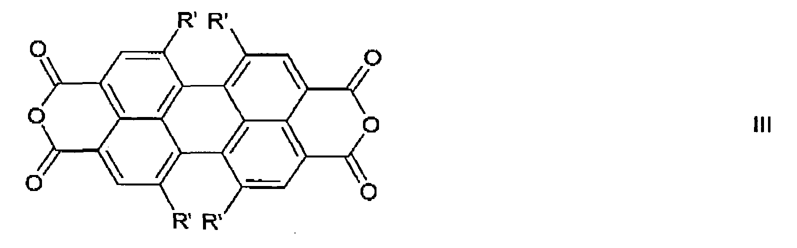 mit einem sterisch gehinderten primären Amin der allgemeinen Formel IV
R-NH₂ IV
in einem im wesentlichen wasserfreien Reaktionsmedium, **dadurch gekennzeichnet, daß** man die Umsetzung in Gegenwart eines tertiären Amins, eines Lösungsmittels auf Basis eines cyclischen Imins oder Amids und einer Lewis-Säure als Katalysator vornimmt.

2. Verfahren nach Anspruch 1, **dadurch gekennzeichnet, daß** man als tertiäres Amin ein Amin der allgemeinen Formel I 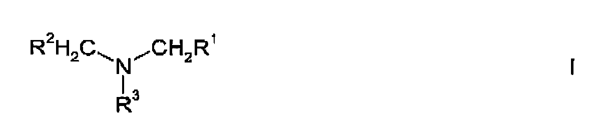 einsetzt, in der die Variablen folgende Bedeutung haben:
R¹, R² unabhängig voneinander
Wasserstoff;
C₁-C₂₃-Alkyl, dessen Kohlenstoffkette durch eine oder mehrere Gruppierungen -O-, -S- und/oder -NR⁴- unterbrochen sein kann und das durch Hydroxy, Cyano, Halogen, Nitro, C₆-C₁₀-Aryl, das ein bis drei Heteroatome im Ringsystem enthalten kann, und/oder C₄-C₁₂-Cycloalkyl, das ein bis vier Heteroatome im Ringsystem enthalten kann, substituiert sein kann;
C₄-C₁₂-Cycloalkyl, dessen Kohlenstoffring durch eine oder mehrere Gruppierungen -O-, -S- und/oder -NR⁴- unterbrochen sein kann und das durch C₁-C₁₈-Alkyl, Hydroxy, Cyano, Halogen und/oder Nitro substituiert sein kann;
C₆-C₁₀-Aryl, das durch C₁-C₁₈-Alkyl, Hydroxy, Cyano, Halogen und/oder Nitro substituiert sein kann;
zusammen einen die Gruppierung -CH₂-NR³-CH₂- enthaltenden 4- bis 9-gliedrigen gesättigten Ring, der durch weitere Gruppierungen -O-, -S-und/oder-NR⁴- unterbrochen sein kann und durch C₁-C₆-Alkyl, C₆-C₁₀-Aryl. Hydroxy, Cyano, Halogen und/oder Nitro subsituiert sein kann;
R³ C₄-C₂₄-Alkyl, dessen Kohlenstoffkette durch eine oder mehrere Gruppierungen -O-, -S- und/oder -NR⁴- unterbrochen sein kann und das durch Hydroxy, Cyano, Nitro, C₆-C₁₀-Aryl, das ein bis drei Heteroatome im Ringsystem enthalten kann, und/oder C₄-C₁₂-Cycloalkyl, das ein bis vier Heteroatome im Ringsystem enthalten kann, substituiert sein kann; C₄-C₁₂-Cycloalkyl, dessen Kohlenstoffring durch eine oder mehrere Gruppierungen -O-, -S- und/oder -NR⁴- unterbrochen sein kann und das durch C₁-C₁₈-Alkyl, Hydroxy, Cyano, Halogen und/oder Nitro substituiert sein kann;
C₆-C₁₀-Aryl, das ein bis drei Heteroatome im Ringsystem enthalten kann und durch C₁-C₁₈-Alkyl, Hydroxy, Cyano und/oder Nitro substituiert sein kann;
R¹, R² und R³ miteinander unter Ausbildung eines gesättigten, bicyclischen, die Gruppierung 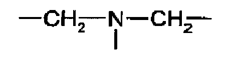 und gegebenenfalls weitere Gruppierungen -O-, -S- und/oder -NR⁴- enthaltenden 8- bis 12-gliedrigen Ringsystems verbunden;
R⁴ Wasserstoff; C₁-C₁₂-Alkyl.

3. Verfahren nach Anspruch 1 oder 2, **dadurch gekennzeichnet, daß** man als tertiäres Amin ein Amin der allgemeinen Formel I 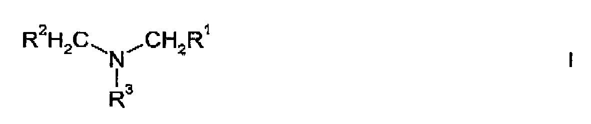 einsetzt, in der die Variablen folgende Bedeutung haben:
R¹, R² unabhängig voneinander
Wasserstoff;
C₁-C₁₁-Alkyl, das durch Hydroxy substituiert sein kann; zusammen einen die Gruppierung -CH₂-NR³-CH₂- enthaltenden 4- bis 9-gliedrigen gesättigten Ring, der durch weitere Gruppierungen -NR⁴- un-. terbrochen sein kann;
R³ C₄-C₁₂-Alkyl, das durch Hydroxy substituiert sein kann; Pyridyl;
R¹, R² und R³ miteinander unter Ausbildung eines bicyclischen, die Gruppierung 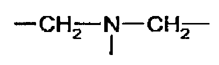 und ein weiteres Stickstoffatom enthaltenden 8-gliedrigen Ringsystems verbunden;
R⁴ Wasserstoff; C₁-C₁₂-Alkyl.

4. Verfahren nach den Ansprüchen 1 bis 3, **dadurch gekennzeichnet, daß** man als tertiäres Amin ein Amin einsetzt, dessen Siedepunkt oberhalb der Reaktionstemperatur liegt.

5. Verfahren nach den Ansprüchen 1 bis 4, **dadurch gekennzeichnet, daß** man als cyclisches Imin ein cyclisches aromatisches Imin einsetzt.

6. Verfahren nach den Ansprüchen 1 bis 5, **dadurch gekennzeichnet, daß** man als Lewis-Säure einen Übergangsmetallkatalysator auf der Basis von Eisen, Zink, Zinksalzen, Kupfer, Kupfersalzen oder Gemischen davon einsetzt.

7. Verfahren nach den Ansprüchen 1 bis 6, **dadurch gekennzeichnet, daß** man 0,01 bis 4 mol tertiäres Amin je mol Perylen-3,4:9,10-tetracarbonsäuredianhydrid einsetzt.

8. Verfahren nach den Ansprüchen 1 bis 7, **dadurch gekennzeichnet, daß** man es zur Herstellung von Perylen-3,4-dicarbonsäureimiden der allgemeinen Formel II 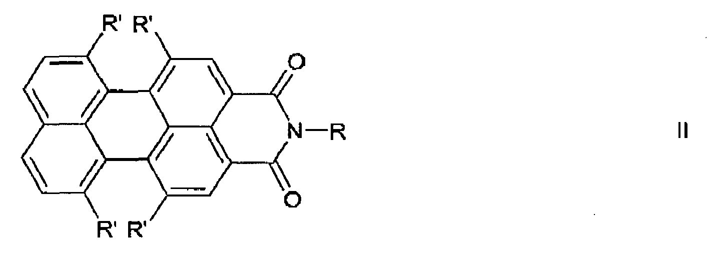 anwendet, in der die Variablen folgende Bedeutung haben:
R C₃-C₂₄-Alkyl, das in 1-Position verzweigt ist; Phenyl, das in beiden ortho-Positionen durch sekundäre C₃-C₁₂-Alkylreste oder in einer ortho-Position durch einen tertiären C₄-C₁₂-Alkylrest substituiert ist und das an den anderen Positionen durch C₁-C₁₂-Alkyl, C₆-C₁₀-Aryl und/oder Halogen substituiert sein kann;
R' Wasserstoff; Halogen; C₆-C₁₀-Aryloxy oder -Arylthio, das jeweils durch C₁-C₁₀-Alkyl, C₁-C₆-Alkoxy, Cyano und/oder Carboxy substituiert sein kann.

## Claims

1. A process for preparing perylene-3,4-dicarboximides which bear a sterically demanding substituent on the imide nitrogen atom and have the general formula II 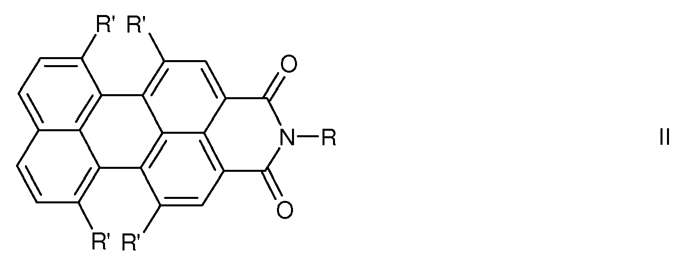 in which the variables are each defined as follows:
R is C₃-C₂₄-alkyl which is branched in the 1-position and whose carbon chain may be interrupted by one or more -0-, -S-, -NR⁴-, -CO-and/or -SO₂- moieties and which may be mono- or polysubstituted by carboxyl, sulfo, hydroxyl, cyano, C₁-C₆-alkoxy, C₃-C₈-cycloalkyl which may comprise from one to four heteroatoms in the ring system and/or C₆-C₁₀-aryl which may comprise from one to three heteroatoms in the ring system;
phenyl which is substituted in the ortho-position by at least one of the substituents (a) to (e) and may bear the same or one or more different substituents (a) to (e) in the further ring positions and which may comprise one or two heteroatoms in the ring:
(a) C₁-C₂₄-alkyl whose carbon chain may be interrupted by one or more -0-, -S-, -NR⁴-, -CO-and/or -SO₂- moieties and which may be mono- or polysubstituted by carboxyl, sulfo, hydroxyl, cyano, halogen, nitro, C₁-C₆-alkoxy, C₃-C₈-cycloalkyl which may comprise from one to four heteroatoms in the ring system and/or C₆-C₁₀-aryl which may comprise from one to three heteroatoms in the ring system;
(b) C₁-C₂₄-alkoxy or C₁-C₂₄-alkylthio, each of which may be mono- or polysubstituted by C₁-C₆-alkyl and/or phenyl;
(c) C₃-C₈-cycloalkyl which may comprise from one to four heteroatoms in the ring system, may comprise unsaturated bonds and may be mono- or polysubstituted by carboxyl, sulfo, hydroxyl, cyano, halogen, nitro, C₁-C₆-alkoxy and/or C₆-C₁₀-aryl which may comprise from one to three heteroatoms in the ring system;
(d) C₆-C₁₀-aryl which may comprise from one to three heteroatoms in the ring system, to which further 5- to 7-membered, saturated, unsaturated or aromatic rings may be fused and which may be mono- or polysubstituted by C₁-C₁₂-alkyl;
(e) carboxyl, hydroxyl, cyano, halogen, nitro, -SO₂NR⁴₂;
1-naphthyl which is substituted in the ortho-position by one of the substituents (a) to (e) specified for phenyl and may bear in the further ring positions the same or one or more different substituents (a) to (e) and which may comprise from one to three heteroatoms in the ring system;
2-naphthyl which is substituted in both ortho-positions by one of the substituents (a) to (e) specified for phenyl or different substituents from this group and may bear in the further ring positions the same or one or more different substituents (a) to (e) and which may comprise from one to three heteroatoms in the ring system;
R' are each independently hydrogen; halogen; C₁-C₁-alkyl; C₆-C₁₀-aryloxy or -arylthio, each of which may comprise from one to three heteroatoms in the ring system and each of which may be substituted by C₁-C₁₀-alkyl, C₁-C₆-alkoxy, cyano and/or carboxyl;
R⁴ is hydrogen; C₁-C₁₂-alkyl,
by reacting a perylene-3,4:9,10-tetracarboxylic dianhydride of the general formula III 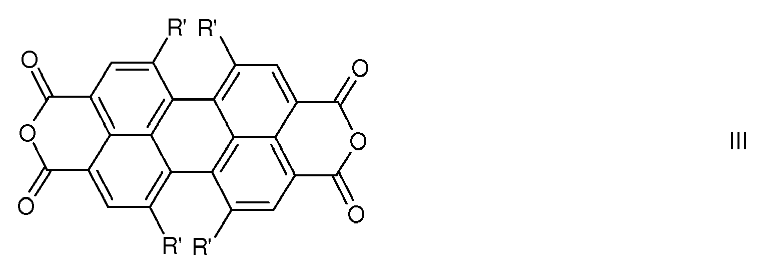 with a sterically hindered primary amine of the general formula IV
R-NH₂ IV
in a substantially anhydrous reaction medium, which comprises undertaking the reaction in the presence of a tertiary amine, of a solvent based on a cyclic imine or amide and of a Lewis acid as a catalyst.

2. The process according to claim 1, wherein the tertiary amine used is an amine of the general formula I 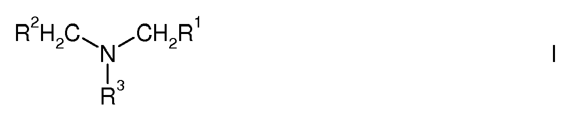 in which the variables are each defined as follows:
R¹, R² are each independently hydrogen;
C₁-C₂₃-alkyl whose carbon chain may be interrupted by one or more -0-, -S- and/or-NR⁴- moieties and which may be substituted by hydroxyl, cyano, halogen, nitro, C₆-C₁₀-aryl which may comprise from one to three heteroatoms in the system and/or C₄-C₁₂-cycloalkyl which may comprise from one to four heteroatoms in the ring system;
C₄-C₁₂-cycloalkyl whose carbon ring may be interrupted by one or more -0-, -S- and/or-NR⁴- moieties and which may be substituted by C₁-C₁₈-alkyl, hydroxyl, cyano, halogen and/or nitro;
C₆-C₁₀-aryl which may be substituted by C₁-C₁₈-alkyl, hydroxyl, cyano, halogen and/or nitro; together are a 4- to 9-membered saturated ring which comprises the -CH₂-NR³-CH₂- moiety and may be interrupted by further -0-, -Sand/or -NR⁴- moieties and may be substituted by C₁-C₆-alkyl, C₆-C₁₀-aryl, hydroxyl, cyano, halogen and/or nitro;
R³ is C₄-C₂₄-alkyl, whose carbon chain may be interrupted by one or more -0-, -S- and/or -NR⁴- moieties and which may be substituted by hydroxyl, cyano, nitro, C₆-C₁₀-aryl which may comprise from one to three heteroatoms in the ring system and/or C₄-C₁₂-cycloalkyl which may comprise from one to four heteroatoms in the ring system;
C₄-C₁₂-cycloalkyl whose carbon ring may be interrupted by one or more -0-, -S- and/or -NR⁴- moieties and which may be substituted by C₁-C₁₈-alkyl, hydroxyl, cyano, halogen and/or nitro;
C₆-C₁₀-aryl which may comprise from one to three heteroatoms in the ring system and which may be substituted by C₁-C₁₈-alkyl, hydroxyl, cyano and/or nitro;
R¹, R² and R³ join together to form a saturated, bicyclic 8- to 12-membered ring system which comprises the 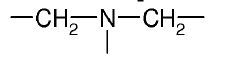 moiety and optionally further -0-, -S- and/or -NR⁴- moieties;
R⁴ is hydrogen; C₁-C₁₂-alkyl.

3. The process according to claim 1 or 2, wherein the tertiary amine used is an amine of the general formula I 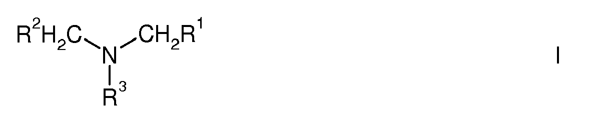 in which the variables are each defined as follows:
R¹, R² are each independently
hydrogen;
C₁-C₁₁-alkyl which may be substituted by hydroxyl;
together are a 4- to 9-membered saturated ring which comprises the
-CH₂-NR³-CH₂- moiety and may be interrupted by further -NR⁴- moieties;
R³ is C₄-C₁₂-alkyl which may be substituted by hydroxyl; pyridyl;
R¹, R² and R³ join together to form a bicyclic 8-membered ring system which comprises the 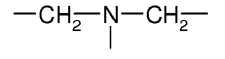 moiety and one further nitrogen atom;
R⁴ is hydrogen; C₁-C₁₂-alkyl.

4. The process according to claims 1 to 3, wherein the tertiary amine used is an amine whose boiling point is above the reaction temperature.

5. The process according to claims 1 to 4, wherein the cyclic imine used is a cyclic aromatic imine.

6. The process according to claims 1 to 5, wherein the Lewis acid used is a transition metal catalyst based on iron, zinc, zinc salts, copper, copper salts or mixtures thereof.

7. The process according to claims 1 to 6, wherein from 0.01 to 4 mol of tertiary amine are used per mole of perylene-3,4:9,10-tetracarboxylic dianhydride.

8. The process according to claims 1 to 7, which is used to prepare perylene-3,4-dicarboximides of the general formula II 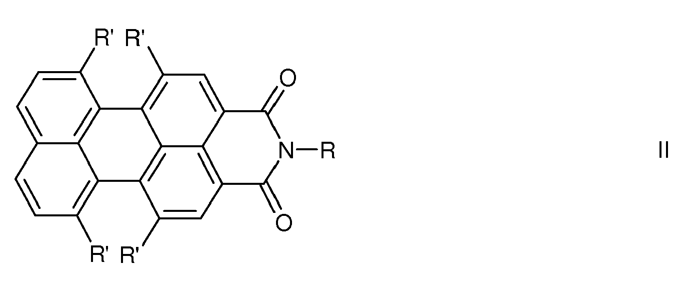 in which the variables are each defined as follows:
R is C₃-C₂₄-alkyl which is branched in the 1-position;
phenyl which is substituted in both ortho-positions by secondary C₃-C₁₂-alkyl radicals or in one ortho-position by a tertiary C₄-C₁₂-alkyl radical and which may be substituted in the other positions by C₁-C₁₂-alkyl, C₆-C₁₀-aryl and/or halogen;
R' is hydrogen; halogen; C₆-C₁₀-aryloxy or-arylthio, each of which may be substituted by C₁-C₁₀-alkyl, C₁-C₆-alkoxy, cyano and/or carboxyl.

## Revendications

1. Procédé pour la préparation de pérylène-3,4-dicarboximides qui portent un substituant à encombrement stérique sur l'atome d'azote en fonction imide et qui présentent la formule générale II 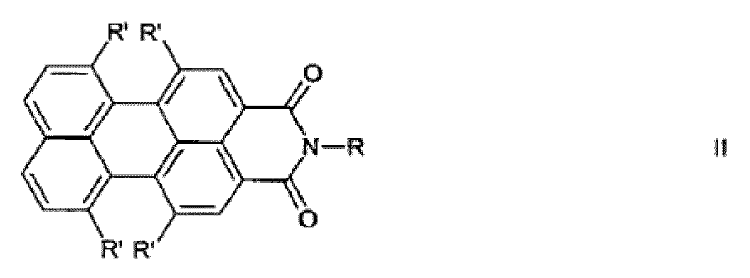 dans laquelle les variables ont la signification suivante :
R représente un groupe alkyle en C₃-C₂₄ qui est ramifié en position 1, dont la chaîne carbonée peut être interrompue par un ou plusieurs groupements -0-, -S-, -NR⁴-, -CO- et/ou -SO₂- et qui peut être une ou plusieurs fois substitué par carboxy, sulfo, hydroxy, cyano, alcoxy en C₁-C₆, cycloalkyle en C₃-C₈ qui peut comporter un à quatre hétéroatomes dans le système cyclique, et/ou aryle en C₆-C₁₀ qui peut comporter un à trois hétéroatomes dans le système cyclique, phényle qui est substitué en une position ortho par au moins un des substituants (a) à (e) et peut porter en les autres positions du cycle les mêmes substituants (a) à (e) ou un ou plusieurs substituants (a) à (e) différents de ceux-ci et qui peut comporter un ou deux hétéroatomes dans le cycle ;
(a) alkyle en C₁-C₂₄, dont la chaîne carbonée peut être interrompue par un ou plusieurs groupements -0-, -S-, -NR⁴-, -CO- et/ou -SO₂- et qui peut être une ou plusieurs fois substitué par carboxy, sulfo, hydroxy, cyano, halogéno, nitro, alcoxy en C₁-C₆, cycloalkyle en C₃-C₈ qui peut comporter un à quatre hétéroatomes dans le système cyclique, et/ou aryle en C₆-C₁₀ qui peut comporter un à trois hétéroatomes dans le système cyclique ;
(b) alcoxy en C₁-C₂₄ ou alkyl(C₁-C₂₄)thio, qui peut dans chaque cas être une ou plusieurs fois substitué par alkyle en C₁-C₆ et/ou phényle ;
(c) cycloalkyle en C₃-C₈ qui peut comporter un à quatre hétéroatomes dans le système cyclique, peut comporter des liaisons insaturées et peut être une ou plusieurs fois substitué par carboxy, sulfo, hydroxy, cyano, halogéno, nitro, alcoxy en C₁-C₆ et/ou aryle en C₆-C₁₀ qui peut comporter un à trois hétéroatomes dans le système cyclique ;
(d) aryle en C₆-C₁₀ qui peut comporter un à trois hétéroatomes dans le système cyclique, auquel peuvent être soudés d'autres cycles saturés, insaturés ou aromatiques à 5 à 7 chaînons et qui peut être une ou plusieurs fois substitué par alkyle en C₁-C₁₂ ;
(e) carboxy, hydroxy, cyano, halogéno, nitro, -SO₂NR⁴₂;
1-naphtyle qui est substitué en position ortho par un des substituants (a) à (e) nommés pour phényle et peut porter en les autres positions du cycle les mêmes substituants (a) à (e) ou un ou plusieurs substituants (a) à (e) différents de ceux-ci et qui peut comporter un à trois hétéroatomes dans le système cyclique ;
2-naphtyle qui est substitué en les deux positions ortho par un ou différents substituants (a) à (e) nommés pour phényle et peut porter en les autres positions du cycle les mêmes substituants (a) à (e) ou un ou plusieurs substituants (a) à (e) différents de ceux-ci et qui peut comporter un à trois hétéroatomes dans le système cyclique ;
R' représente chaque fois indépendamment un atome d'hydrogène ; un atome d'halogène ; un groupe alkyle en C₁-C₁₈ ; un groupe aryl(C₆-C₁₀)oxy ou aryl(C₆-C₁₀)thio, qui peut dans chaque cas comporter un à trois hétéroatomes dans le système cyclique et qui peut dans chaque cas être substitué par alkyle en C₁-C₁₀, alcoxy en C₁-C₆, cyano et/ou carboxy ;
R⁴ représente un atome d'hydrogène ; un groupe alkyle en C₁-C₁₂,
par mise en réaction d'un dianhydride d'acide pérylène-3,4:9,10-tétracarboxylique de formule générale III 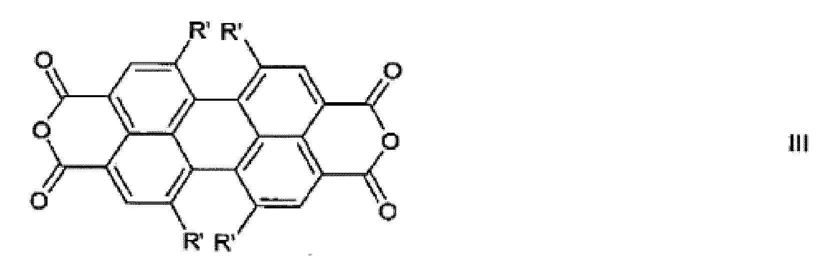 avec une amine primaire à empêchement stérique de formule générale IV
R-NH₂ IV
dans un milieu réactionnel essentiellement anhydre, **caractérisé en ce qu'**on effectue la réaction en présence d'une amine tertiaire, d'un solvant à base d'une imine cyclique ou d'un amide cyclique et d'un acide de Lewis en tant que catalyseur.

2. Procédé selon la revendication 1, **caractérisé en ce qu'**on utilise comme amine tertiaire une amine de formule générale I 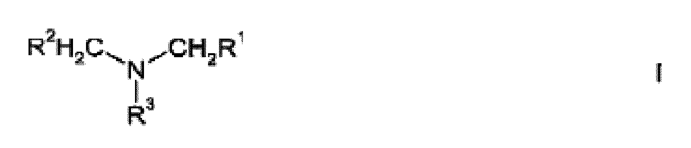 dans laquelle les variables ont la signification suivante :
R¹, R² indépendamment l'un de l'autre représentent un atome d'hydrogène ; un groupe alkyle en C₁-C₂₃, dont la chaîne carbonée peut être interrompue par un ou plusieurs groupements -0-, -S- et/ou -NR⁴- et qui peut être substitué par hydroxy, cyano, halogéno, nitro, aryle en C₆-C₁₀ qui peut comporter un à trois hétéroatomes dans le système cyclique, et/ou cycloalkyle en C₄-C₁₂ qui peut comporter un à quatre hétéroatomes dans le système cyclique ;
un groupe cycloalkyle en C₄-C₁₂, dont le cycle carboné peut être interrompu par un ou plusieurs groupements -0-, -S- et/ou -NR⁴- et qui peut être substitué par alkyle en C₁-C₁₈, hydroxy, cyano, halogéno et/ou nitro ;
un groupe aryle en C₆-C₁₀ qui peut être substitué par alkyle en C₁-C₁₈, hydroxy, cyano, halogéno et/ou nitro ;
ensemble forment un cycle saturé à 4 à 9 chaînons, contenant le groupement -CH₂-NR³-CH₂-, qui peut être interrompu par d'autres groupements -0-, -S- et/ou -NR⁴- et peut être substitué par alkyle en C₁-C₆, aryle en C₆-C₁₀, hydroxy, cyano, halogéno et/ou nitro ;
R³ représente un groupe alkyle en C₄-C₂₄, dont la chaîne carbonée peut être interrompue par un ou plusieurs groupements -0-, -S- et/ou -NR⁴- et qui peut être substitué par hydroxy, cyano, nitro, aryle en C₆-C₁₀ qui peut comporter un à trois hétéroatomes dans le système cyclique, et/ou cycloalkyle en C₄-C₁₂ qui peut comporter un à quatre hétéroatomes dans le système cyclique ; un groupe cycloalkyle en C₄-C₁₂, dont le cycle carboné peut être interrompu par un ou plusieurs groupements -0-, -S- et/ou -NR⁴- et qui peut être substitué par alkyle en C₁-C₁₈, hydroxy, cyano, halogéno et/ou nitro ;
un groupe aryle en C₆-C₁₀ qui comporter un à trois hétéroatomes dans le système cyclique et peut être substitué par alkyle en C₁-C₁₈, hydroxy, cyano et/ou nitro ;
R¹, R² et R³ sont liés entre eux avec formation d'un système cyclique saturé, bicyclique, à 8 à 12 chaînons formant le cycle, contenant le groupement 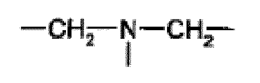 et éventuellement d'autres groupements -O-,-S-et/ou -NR⁴- ;
R⁴ représente un atome d'hydrogène, un groupe alkyle en C₁-C₁₂.

3. Procédé selon la revendication 1 ou 2, **caractérisé en ce qu'**on utilise comme amine tertiaire une amine de formule générale I 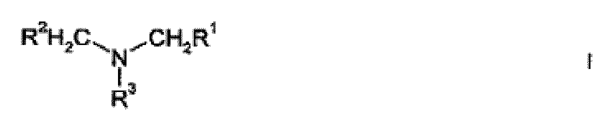 dans laquelle les variables ont la signification suivante :
R¹, R² indépendamment l'un de l'autre représentent un atome d'hydrogène ;
un groupe alkyle en C₁-C₁₁ qui peut être substitué par hydroxy ;
ensemble forment un cycle saturé à 4 à 9 chaînons, contenant le groupement -CH₂-NR³-CH₂-, qui peut être interrompu par d'autres groupements -NR⁴- ;
R³ représente un groupe alkyle en C₄-C₁₂ qui peut être substitué par hydroxy ; le groupe pyridyle ;
R¹, R² et R³ sont liés entre eux avec formation d'un système cyclique, bicyclique, à 8 chaînons, contenant le groupement 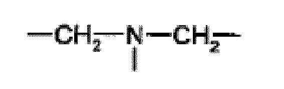 et un autre atome d'azote ;
R⁴ représente un atome d'hydrogène, un groupe alkyle en C₁-C₁₂.

4. Procédé selon les revendications 1 à 3, **caractérisé en ce qu'**on utilise comme amine tertiaire une amine dont le point d'ébullition se situe au-dessus de la température de la réaction.

5. Procédé selon les revendications 1 à 4, **caractérisé en ce qu'**on utilise comme imine cyclique une imine cyclique aromatique.

6. Procédé selon les revendications 1 à 5, **caractérisé en ce qu'**on utilise comme acide de Lewis un catalyseur à métal de transition à base de fer, zinc, sels de zinc, cuivre, sels de cuivre ou mélanges de ceux-ci.

7. Procédé selon les revendications 1 à 6, **caractérisé en ce qu'**on utilise 0,01 à 4 moles d'amine tertiaire par mole d'anhydride d'acide pérylène-3,4:9,10-tétracarboxylique.

8. Procédé selon les revendications 1 à 7, **caractérisé en ce qu'**on l'utilise pour la préparation de pérylène-3,4-dicarboximides de formule générale II 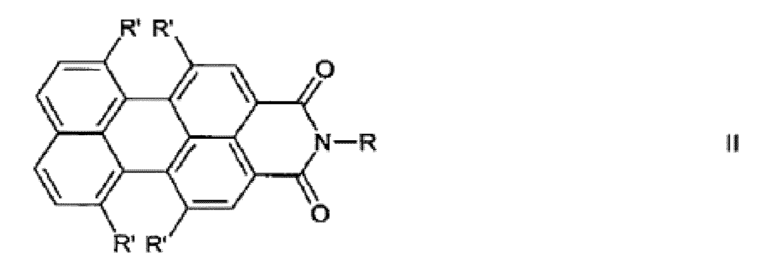 dans laquelle les variables ont la signification suivante :
R représente un groupe alkyle en C₃-C₂₄ qui est ramifié en position 1,
un groupe phényle qui est substitué en les deux positions ortho par des radicaux alkyle en C₃-C₁₂ secondaires ou en une position ortho par un radical alkyle en C₄-C₁₂ tertiaire et qui peut être substitué en les autres positions par alkyle en C₁-C₁₂, aryle en C₆-C₁₀ et/ou halogéno ;
R' représente un atome d'hydrogène ; un atome d'halogène ; un groupe aryl(C₆-C₁₀)oxy ou aryl(C₆-C₁₀)thio, qui peut dans chaque cas être substitué par alkyle en C₁-C₁₀, alcoxy en C₁-C₆, cyano et/ou carboxy.
